(19) Europäisches Patentamt
European Patent Office
Office européen des brevets

(11) Publication number: **0 008 767**
**B1**

(12) **EUROPEAN PATENT SPECIFICATION**

(45) Date of publication of patent specification: **04.11.81**

(51) Int. Cl.³: **C 07 C 29/14, C 07 C 31/02**

(21) Application number: **79103181.8**

(22) Date of filing: **28.08.79**

# (54) A heterogeneous vapor phase process for the catalytic hydrogenation of aldehydes to alcohols.

(30) Priority: **29.08.78 US 937713**

(43) Date of publication of application:
**19.03.80 Bulletin 80/6**

(45) Publication of the grant of the European patent:
**04.11.81 Bulletin 81/44**

(84) Designated Contracting States:
**BE DE FR GB IT NL**

(56) References cited:
**DE - C - 1 226 083**
**US - A - 2 079 414**
**US - A - 2 549 416**
**US - A - 4 052 467**

(73) Proprietor: **UNION CARBIDE CORPORATION**
**270, Park Avenue**
**New York, N.Y. 10017 (US)**

(72) Inventor: **Pai, Chao-Chyan**
**11523 Sageking Drive**
**Houston Texas (US)**

(74) Representative: **Schmied-Kowarzik, Volker, Dr. et al,**
**Patentanwälte Dipl.-Ing. P. Wirth Dr. V. Schmied-Kowarzik Dipl.-Ing. G. Dannenberg, Dr. P. Weinhold Dr. D. Gudel, Dipl.-Ing. S. Schubert**
**Siegfriedstrasse 8 D-8000 München 40 (DE)**

**EP 0 008 767 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

A heterogeneous vapor phase process for the catalytic hydrogenation of aldehydes to alcohols

This invention relates to a heterogeneous vapor phase process for the catalytic hydrogenation of aldehyde(s) containing 2 to 8 carbon atoms to the corresponding alcohol(s) by contacting a vaporous stream of aldehyde(s) and a hydrogen containing gas with a solid catalyst comprising a reduced mixture of CuO and ZnO which is characterized by carrying out the reaction at a temperature of between 110°C and 180°C, a pressure of between 2,4 and 11,5 bar (20 and 150 psig) and at a space velocity of 500 and 4000 $hr^{-1}$.

It is well known to hydrogenate aldehydes to produce alcohols. The hydrogen adds onto the carbonyl group of the aldehyde to form the alcohol. The aldehyde so hydrogenated, may be a mixture of aldehydes formed by the oxo process, i.e., the reaction of olefins with carbon monoxide and hydrogen in the presence of a catalyst to add a carbonyl group at one of the carbon atoms of the olefinic group.

The reaction of aldehyde and hydrogen is carried out in the presence of certain metal compounds which act as hydrogenation catalysts. The commonly used commercial hydrogenation catalysts include copper chromite; cobalt compounds; nickel; nickel compounds which may contain small amounts of chromium or other promoters; and mixtures of copper and nickel and/or chromium. The nickel compounds generally are deposited on support materials such as alumina and kieselguhr. Nickel catalysts are believed to be the most commonly used hydrogenation catalysts for converting aldehydes to alcohols, because of their apparent activity.

There are one or more disadvantages in the use of these hydrogenation catalysts in a commercial operation catalytically hydrogenating aldehydes to alcohols.

The use of copper chromite as an aldehyde hydrogenation catalyst requires that the hydrogenation process be carried out at higher temperatures and pressures. Also, since copper catalysts are easily poisoned by sulfur compounds, costly and stringent controls of the process are required to remove and prevent these sulfur compounds from entering into the hydrogenation reactor. Additionally, the use of copper chromite catalysts is environmentally hazardous and requires special and costly handling techniques because of the toxicity of the chromium. Lastly, the preparation of copper chromite is difficult and reproducibility in its manufacturing process is poor. However, copper chromite catalysts are still used as aldehyde hydrogenation catalysts because of their high selectivity.

Cobalt compounds are active aldehyde hydrogenation catalysts but their use requires that the hydrogenation process be carried out at higher temperatures and pressures. Also, their activity is lower and they are more costly (cobalt supply is becoming scarce at the present time) than either nickel catalyst or copper chromite catalysts. Further, the selectivity of cobalt catalysts is not as high as that of copper chromite.

Nickel catalysts are highly active aldehyde hydrogenation catalysts. An aldehyde hydrogenation process using nickel catalysts requires lower reaction temperatures and pressures than the use of either copper chromite or cobalt catalysts, a factor which makes them the most commonly used commercial hydrogenation catalysts. However, there are disadvantages in the use of these nickel catalysts in a commercial aldehyde hydrogenation process. A disadvantage of the nickel catalysts is that they are environmentally hazardous and require special and costly handling techniques because of their toxicity.

A major and costly disadvantage in using nickel catalysts, as well as several of the other types of hydrogenation catalysts, is the high level of by-products formed when hydrogenating aldehydes to alcohols. For most end use applications, the by-products must be separated from the hydrogenation product.

The principal by-products are ethers and hydrocarbon gases. The amount of ethers formed may be anywhere from 0.5 to 2.0 weight percent and higher, based on the reaction product. Since ethers form an azeotrope with the alcohol products formed in the aldehyde hydrogenation process and water, which is frequently present in the product from the feed streams, a substantial amount of energy is required to separate the ether from the alcohol. For example, in the catalytic hydrogenation of butyraldehyde to butanol, butyl ether forms an azeotrope with the butanol product and water present therein. Butyl ether must be separated from the butanol, in order for butanol to pass purity specifications such as the specifications for use in making acrylates. This separation of butyl ether from butanol requires a series of costly distillation steps, and because of the butyl ether-butanol azeotrope, four kilogrammes of butanol is lost for every kilogramme of butyl ether formed.

The by-product hydrocarbon gases are produced by the decomposition of aldehydes. For example, propionaldehyde is decomposed according to the following equation:

$$CH_3CH_2CHO + 3H_2 \rightarrow CH_3CH_3 + CH_4 + H_2O$$

This reaction results in aldehyde losses of anywhere from1 to 5 percent, because of the reaction of aldehyde (propionaldehyde) and hydrogen to form the hydrocarbon gases (methane and ethane). Also, this reaction causes losses in hydrogen efficiency of

anywhere from 20 to 50 percent. (Hydrogen efficiency = the stoichiometric amount of hydrogen required in the reaction/actual amount of hydrogen fed to the system). Loss in hydrogen efficiency is caused by the heavier hydrocarbon gases building up in the hydrogen recycle stream, which necessitates a need to increase the purge stream of the recycle line to avoid overloading the cycle compressor and to maintain hydrogen purity.

Thus, the elimination of the formation of by-product ethers and/or hydrocarbon gases in a commercial aldehyde hydrogenation process is highly desirable, resulting in less energy consumption and a reduction in the investment in distillation equipment. Additionally, hydrogen and aldehyde efficiencies are increased by eliminating the formation of these by-products.

A reduced mixture of the oxides of Cu and Zn has been used as a catalyst for reducing aldehydes to alcohols. U.S. Patent 2,549,416 describes a vapor phase process for hydrogenating aldehydes by passing them together with hydrogen over a catalyst comprising reduced copper plus zinc oxide. The process is carried out at temperatures of 100° to 300°C and pressure of at least one bar (one atmosphere). The preferred temperature range is 150° to 250°C while pressures of about 15 to 43 bar (200 to 600 psig) are preferred.

U.S. Patent 4,052,467 describes a process for hydrogenating aldehydes to alcohols wherein the liquid feed contains at least 10 ppm and up to 1 weight percent of sulfur in the form of ring-type sulfur compounds. The liquid feed in admixture with hydrogen is contacted with a catalyst comprising a reduced mixture of oxides or hydroxides of Cu and Zn at a temperature of 232 to 287°C (450—550°F) and pressure of about 57 to 85 bar (800 to 1200 psig).

However, when the hydrogenation of aldehydes is carried out using a reduced mixture of CuO and ZnO as catalyst, temperature greater than 180°C and pressure greater than 11,5 bar (150 psig), a considerable amount of "heavies", such as esters are formed. For example, in the catalytic hydrogenation of propionaldehyde to propanol using said catalyst and reaction conditions, propyl propionate is formed. The propyl propionate must be separated from the propanol which requires a costly separation procedure.

A process has now been found providing unobvious advantages over conventional hydrogenation processes. In accordance with the process of this invention aldehyde(s) containing 2 to 8 carbon atoms can be catalytically hydrogenated to alcohol(s) with negligible formation of by-products such as ethers, and hydrocarbon gases and also, with small amounts of "heavies" formation. The process involves contacting a vaporous stream of aldehyde(s) and hydrogen-containing gas with a solid catalyst comprising a reduced mixture of CuO and ZnO at a temperature of between 110° and 180°C, a pressure of between 2,4 and 11,5 bar (20 and 150 psig) and a space velocity of 500 and 4000 $hr^{-1}$.

With the use of a reduced mixture of CuO and ZnO as the catalyst in a process for hydrogenating aldehyde(s) containing 2 to 8 carbon atoms to alcohol(s) and with the synergistic combination of reaction temperatures of between 110° and 180°C and pressures of between 2,4 and 11,5 bar (20 and 150 psig), the formation of by-product ether and hydrocarbon gases is negligible and the formation of "heavies" is low. This results in less energy consumption in the process since the refining system used to remove these by-products and "heavies" from the alcohol product can be eliminated or greatly simplified. Moreover, hydrogen efficiency using the catalyst and reaction conditions of the present invention is increased to over 90 percent and aldehyde efficiencies are also high. (Aldehyde efficiency = actual amount of alcohol produced in the reaction/the stoichiometric amount of alcohol that would be produced). Additionally, high aldehyde conversions (as high as 99.99% conversions) and high alcohol yields (over 99% alcohol yield) may be achieved by the use of the catalyst and reaction conditions of the present invention. (Aldehyde conversion = amount of aldehyde converted/total amount of aldehyde in the feed; Alcohol yield = amount of aldehyde converted to alcohol/total amount of aldehyde in the feed). Moreover, the present catalysts have several advantages over copper chromite hydrogenation catalysts, such as their resistance to poisoning by sulfur compounds, their high reproducibility in manufacture, their lower manufacturing costs, their low environmental hazards and long life. Also, the activity of the present catalysts is generally about forty percent higher than the activity of copper chromite catalysts as determined on the basis of equal copper content. The process of the present invention is suitable for hydrogenating straight or branched aldehyde(s) containing 2 to 8 carbon atoms. These aldehydes include saturated aldehydes like acetaldehyde, propionaldehyde, isobutyraldehyde, n-butyraldehyde, isopentanal, n-pentanal, 2-methylpentanal, crotonaldehyde, 2-ethylhexanal, 2-ethylbutyraldehyde, and unsaturated $C_{3-8}$-aldehydes like acrolein, 2-ethyl-3-propylacrolein.

$$(CH_3CH_2CH_2CH{=}\overset{\overset{\displaystyle C_2H_5}{|}}{C}{-}CHO).$$

The aldehyde may be in a substantially pure state or admixed with a component(s) other than an aldehyde. Further, a mixture of aldehydes may be employed.

The aldehyde or mixture of aldehydes employed herein may be obtained by an oxo process. A portion of the totality of the product mixture of an oxo process may be employed.

Thus, the aldehyde(s) products or a portion of them may be separated from the product stream of an oxo process for hydrogenation by the process of this invention. For the purpose of providing an aldehyde feed, a conventional oxo product stream may be employed.

The aldehyde or mixture of aldehydes employed herein may also be obtained by processes other than the oxo process, such as by oxidation of olefins or saturated hydrocarbons or by an aldol condensation.

The process of the present invention comprises contacting a vaporous stream of aldehyde(s) containing 2 to 8 carbon atoms and hydrogen alone or in admixture with other gases (desirably gases inert to the aldehyde and the catalyst), with a solid catalyst comprising a reduced mixture of CuO and ZnO. The gaseous mixtures containing hydrogen include inert gases such as nitrogen, or carbon dioxide.

The term "hydrogen-containing gas" as used herein includes both substantially pure hydrogen gas as well as gaseous mixtures containing hydrogen.

The mole ratio of contained hydrogen gas to aldehyde(s) may be generally from 15 to 40 and preferably, from 20 to 30.

The hydrogenation process of the present invention is conducted at a temperature of between 110 and 180°C preferably between 130° and 170°C and at a pressure of between 2,4 and 11,5 bar (20 and 150 psig), preferably between 4,5 and 8 bar (50 and 100 psig).

The mixture of CuO and ZnO, before reduction, contains from 10 to 70 percent by weight CuO and from 90 to 30 percent by weight of ZnO. The preferred amount of CuO in the mixture is from 20 to 40 percent by weight, while the preferred amount of ZnO in the mixture is from 60 to 80 percent by weight. The most preferred amount of CuO in the mixture is from 30 to 36 percent by weight while the most preferred amount of ZnO in the mixture is from 62 to 68 percent by weight. The catalyst may contain minor amounts of other materials such as chloride, sodium, sulfur and aluminum oxide. The catalyst may be prepared by any of the methods known in the art of forming a composite of copper oxide and zinc oxide. The catalyst may be prepared by fixing the separate oxides, by coprecipitation of the oxylates, carbonates, or acetates, followed by calcination. The coprecipitation method is preferred. Generally, the mixture of CuO and ZnO is reduced by hydrogen or carbon monoxide at a temperature in the range of between 160° and 250°C for several hours, preferably for 8 to 24 hours.

The mixture of CuO and ZnO is reduced prior to its use as catalyst in the aldehyde hydrogenation process. Hydrogen or CO, or mixtures thereof, are used as the reducing agent. The hydrogen, CO, or mixtures thereof, are generally mixed with an inert gas such as steam, nitrogen, or combustion gas, to maintain the catalyst bed temperature and to carry away the heat of reduction.

Reduction of the mixture of CuO and ZnO is complete when no more hydrogen is being reacted as shown by analysis of the inlet and outlet hydrogen. Complete reduction of the mixture occurs when the total amount of water produced in the reduction is equal to the stoichiometric value of water which should be produced when a given amount of copper oxide is reduced to copper. This value is about 0.079 kg of water per kg of catalyst for a mixture containing 35 weight percent of CuO.

An inert carrier material may be included in the catalyst composition. The catalyst is generally formed into pellets, tablets, or any other suitable shape prior to use, by conventional techniques.

It is advantageous that the mixture of CuO and ZnO have an internal surface area of 25 to 40 $m^2$ per gram. The internal surface area is determined by the well-known BET method.

The process of the present invention is most conveniently carried out in a continuous manner, although semi-continuous or batch operations may also be employed. In the preferred method of continuous operation, an aldehyde, mixture of aldehydes, or oxo reaction products, a hydrogen containing gas, and optionally, a carrier gas such as nitrogen, may be brought together and, under the desired pressure contacted in the vaporous state with the catalyst. The reaction zone advantageously is an elongated tubular reactor wherein the catalyst is positioned. The vapors are contacted with the catalyst.

The present process is preferably carried out at a space velocity of between 1000 and 4000 $hr^{-1}$ (space velocity is total volume of aldehyde and hydrogen at standard conditions of temperature and pressure per volume of the catalyst per hour).

The alcohol product from the hydrogenation reaction is separated from the hydrogen by condensation and the excess hydrogen is compressed and recycled to the reaction zone. The crude alcohol product may be used in this form or it can be further purified in a conventional manner such as, by fractional distillation. If desired, any unconverted portion of the aldehyde or aldehyde mixture may be separated from the reaction product and recycled to the reaction zone and preferably, admixed with fresh feed gases prior to entering the reaction zone.

The following examples are merely illustrative and are not presented as a definition of the limits of the invention.

### Example 1

This Example illustrates a large scale test utilizing a reduced mixture of CuO and ZnO as catalyst to hydrogenate propionaldehyde to the corresponding alcohol, without the formation of by-product ethers or hydrocarbon gases. The

procedure employed was as follows: 22,6 $m^3$ (800 cubic feet) of a mixture of CuO and ZnO containing 33±3 percent CuO and 65±3 percent ZnO in the form of 6,3 mm (1/4 inch) tablets was charged to a tube-and-shell reactor with 8,26 cm (3.25 inch) outside diameter tubes having an inside diameter of 7,6 cm (3 inches). The mixture of CuO and ZnO was reduced in the reactor by contacting it with hydrogen gas (about 1 to 10 volume percent) in a nitrogen carrier gas, at a space velocity of 400 $hr^{-1}$ and temperature of between 156° and 220°C for eight hours. This reduced mixture of CuO and ZnO was contacted in the reactor with vaporized 99.9 weight percent propionaldehyde, on a water free basis, at a rate of between 2268 and 4763 kg/hr (5000 and 10,500 lb/hr). A typical test was conducted at a propionaldehyde feed rate of 3266 kg/hr (7200 lb/hr) and hydrogen make-up rate of 1358 $m^3$ (48,000 standard cubic feet) per hour (SCFH). The operating conditions were 124°C shell temperature, 113°C inlet temperature, 4,5 bar (50 psig) system pressure, 35 900 $m^3$ (1.27MM SCFH) cycle flow rate and 87 percent $H_2$ cycle purity. A peak temperature of 150°C was observed in the reactor. The reaction product was collected in a condenser and analyzed. The reaction product had the following composition: 99.81 weight percent propanol, 0.08 weight percent propyl propionate, 0.03 weight percent 2-methylpentanol and unknowns, excluding 0.93 weight percent $H_2O$ which came primarily from the feed. No ethers were detected in the product. Only 0.62 volume percent of ethane gas was found in the cycle gas, which came from the hydrogenation of ethylene dissolved in the propionaldehyde feed.

Example 2

This Example illustrates a large scale test utilizing a reduced mixture of CuO and ZnO as catalyst to hydrogenate 2-methylbutyraldehyde to the corresponding alcohol without formation of by-product ethers or hydrocarbon gases.

The catalyst, catalyst reduction procedure and reactor were the same as in Example 1.

The reduced mixture of CuO and ZnO was contacted with 99.0 weight percent of vaporized 2-methyl-butyraldehyde at a rate of 5534 kg/hr (12,200 lb/hr) and 99.0 volume percent $H_2$ make-up gas at a rate of 1811 $m^3$ (64,000 SCFH). The operating conditions were 135°C shell temperature, 117°C inlet temperature, 5,3 bar (61 psig) system pressure, 33 100 $m^3$ (1.17MM SCFH) cycle flow rate and 96.7 volume percent $H_2$ cycle purity. A peak temperature of 161°C was observed in the reactor. The reaction product was collected in a condenser and analyzed. The reaction product had the following composition: 99.02 weight percent 2-methylbutanol and 0.13 weight percent residues. No ethers were detected in the product. Only 0.05 volume percent of butane gas was found in the cycle gas which came from the hydrogenation of butylene dissolved in the aldehyde feed.

Example 3

This Example illustrates a large scale test utilizing a reduced mixture of CuO and ZnO as catalyst to hydrogenate a mixture of iso-pentanal and n-pentanal to the corresponding alcohols without formation of by-product ethers or hydrocarbon gases. The catalyst, catalyst reduction procedure and reactor were the same as in Example 1. The reduced mixture of CuO and ZnO was contacted with a mixture of 31.42 weight percent of vaporized iso-pentanal and 66.44 weight percent of vaporized n-pentanal at a rate of 5489 kg/hr (12,100 lb/hr) and 99 volume percent $H_2$ make-up at a rate of 1783 $m^3$ (63,000 SCFH). The operating conditions were 128°C shell temperature, 113°C inlet temperature, 5,2 bar (60 psig) system pressure and 38 200 $m^3$ (1.35MM SCFH) cycle flow rate. A peak temperature of 143°C was observed in the reactor. The reaction product was collected in a condenser and analyzed. The reaction product had the following composition: 28.91 weight percent iso-pentyl alcohol and 68.70 weight percent n-pentyl alcohol. No ethers or hydrocarbon gases were detected in the product.

Example 4

This Example illustrates a large scale test utilizing a reduced mixture of CuO and ZnO as catalyst to hydrogenate a mixed butyraldehyde product from an oxo process, to the corresponding alcohols without formation of by-product ethers or hydrocarbon gases. The procedure employed was as follows 39,6 $m^3$ (1400 cubic feet) of a mixture of CuO and ZnO containing 33±3 percent CuO and 65±3 percent ZnO in the form of 6,4 x 6,3 mm (1/4 inch x 1/4 inch) pellets was charged to two tube-and-shell reactors in parallel with 8,26 cm (3,25 inch) outside diameter tubes having an inside diameter of 7,6 cm (3 inches). The mixture of CuO and ZnO was reduced as in Example 1. The reduced mixture of ZnO and CuO was contacted with a mixture of 15.07 weight percent vaporized iso-butyraldehyde, 84.03 weight percent vaporized n-butyraldehyde and 0.4 weight percent $H_2O$ at a rate of 8317 kg/hr (18,336 lb/hr) and 99.9 volume percent $H_2$ make-up at 240 kg/hr (529 lb/hr). The operating conditions were 117°C shell temperature, 113°C inlet temperature, 5,2 bar (60 psig) system pressure and 7598,7 kg/hr (16752 lb/hr) cycle flow rate. A peak temperature of 167°C was observed in the reactor. The reaction product was collected in a condenser and analyzed. The reaction product had the following composition: 17.57 weight percent iso-butanol, 81.33 weight percent n-butanol, 0.10 weight percent butyl butyrate, 0.09 weight percent 2-ethylhexanol and 0.74 weight percent $H_2O$. No ethers or hydrocarbon

gases were detected in the product. Ester grade butanol was made with high efficiency and low steam usage by passing the product through a refining system.

Example 5

This Example illustrates a large scale test utilizing the same catalyst, catalyst reduction procedure and reactor as those described in Example 1, to hydrogenate 2-ethyl-3-propylacrolein from an aldol condensation process, to the corresponding alcohol without formation of by-product ethers or hydrocarbon gases.

The procedure employed was as follows: The reduced mixture of CuO and ZnO was contacted with 95.78 weight percent of vaporized 2-ethyl-3-propylacrolein at a rate of 3629 kg/hr (8,000 lb/hr) and 98 volume percent of $H_2$ make-up at 2151 $m^3$ (76,000 SCFH). The process was operated at 147°C shell temperature, 159°C inlet temperature, 5,3 bar (61 psig) system pressure and 31 100 $m^3$ (1.1MM SCFH) cycle flow rate. The resulting product was collected in a condenser and analyzed. The reaction product had the following composition: 95.83 weight percent 2-ethylhexanol, 0.06 weight percent 2-ethyl-3-propylacrolein and 0.16 weight percent 2-ethyl-hexanal. No ethers or hydrocarbon gases were detected in the product.

Comparative Example 6

The procedure employed in these experiments was as follows: A Berty bottom stirred back-mixed differential reactor was used (this reactor was constructed according to the procedure as set forth in J.M. Berty "Reactor for Vapor-Phase Catalytic Studies," Chemical Engineering Progress, Vol. 70, No. 5, May, 1974). The Reactor was charged with 64 ml of the catalysts as set forth in Table I. 99.8 weight percent propionaldehyde and 99.9 volume percent hydrogen were fed to a vaporizer at the rate 120 ml/hr and 0,17 $m^3$ (6 SCFH) respectively. The vaporized aldehyde and hydrogen were red to the Berty reactor operated at a pressure of 5,2 bar (60 psig) and temperature of 160°C. The product vapor was withdrawn from the reactor and fed to a brine cooled condenser and the gas and liquid separated. Both liquid product and vent gas were collected and analyzed by a gas chromatograph.

The composition of the catalysts, and optionally, support material, and analysis of the reaction product are set forth in the Table.

The data in the Table illustrates that the use of the hydrogenation catalyst of the present invention (Experiment 1) is highly selective to propanol with high yields of propanol without formation of by-product ethers or hydrocarbon gases, as compared to the known commercially available aldehyde hydrogenation catalysts.

TABLE

| Experiment | 1 [b] | 2 | 3 | 4 | 5 | 6 | 7 | 8 | 9 | 10 | 11 |
|---|---|---|---|---|---|---|---|---|---|---|---|
| Catalyst Components | CuO 35%<br>ZnO 65% | Ni 16% | Ni 25% | Ni 40% | Ni 60% | Ni 36% | Cu 31%<br>Cr 25%<br>Ba 10% | Ni 50%<br>Zr 2% | Ni 11% | Co 34.5% | Ni 40% |
| Support | None | α-Alumina | Refractory | α-Alumina | α-Alumina | $SiO_2/Al_2O_3$ | None | $SiO_2$ | Ceramic | Refractory Oxide | Refractory |
| Production Composition Wt Percent | | | | | | | | | | | |
| $H_2O$ | 0.16 | 1.26 | 0.52 | 2.09 | 1.00 | 1.23 | 0.68 | 7.70 | – | 1.31 | 2.60 |
| Propionaldehyde | 9.22 | 18.90 | 35.68 | 8.38 | 8.44 | 23.44 | 14.66 | 8.85 | 37.61 | 7.76 | 6.38 |
| Propanol | 90.33 | 75.90 | 63.45 | 83.63 | 88.59 | 73.95 | 84.59 | 56.68 | 61.92 | 90.61 | 86.03 |
| Propyl Ether | 0 | 3.20 | 0 | 5.53 | 1.66 | 1.38 | 0 | 23.18 | 0.04 | 0.17 | 4.16 |
| Heavies [a] | 0.26 | 0.64 | 0.34 | 0.35 | 0.38 | 0.08 | 0.24 | 0.28 | 0.36 | 0.09 | 0.12 |
| Mol % Ethane in vent gas | 0 | 0.23 | 0.31 | 0.50 | 0.44 | 0.27 | 0 | 2.78 | 0.15 | 0.14 | 0.91 |

(a) – Primarily propyl propionate.

(b) – Unreduced form. The mixture of ZnO and CuO was reduced prior to its use by contacting it with hydrogen (1 to 10 weight percent) in a nitrogen carrier gas at a space velocity of 400 $hr^{-1}$ and temperature of between 156° and 220°C for eight hours.

Example 7

A Berty bottom stirred back-mixed differential reactor as described in Example 6 was charged with 70.1 grams of a mixture of CuO (66.8 percent) and ZnO (33.2 percent) in the form of 6,3 x 6,3 mm (1/4 inch x 1/4 inch) tablets. 99.8 weight percent butyraldehyde and 99.9 volume percent hydrogen were fed to a vaporizer at the rate of 131 ml/hr and 0,535 $m^3$ (18.9 SCFH) respectively. The vaporized aldehyde and hydrogen were fed to the Berty reactor operated at a pressure of 4,6 bar (52 psig) and temperature of 161°C. The product vapor was withdrawn from the reactor and fed to a brine cooled condenser and the gas and liquid separated. Both liquid product and vent gas were collected and analyzed by a gas chromatograph. The reaction product had the following composition: 17.5 weight percent butyraldehyde, 82.0 weight percent butanol, 0.32 weight percent butyl butyrate and 0.16 weight percent 2-ethylhexanol. No ethers or hydrocarbon gases were formed.

Example 8

A Berty bottom stirred back-mixed differential reactor as described in Example 6 was charged with 88.5 grams of a mixture of CuO (33±3 percent) and ZnO (65±3 percent) in the form of 6,3 x 6,3 mm (1/4 inch x 1/4 inch) tablets. 99.8 weight percent butyraldehyde and 99.9 volume percent hydrogen were fed to a vaporizer at the rate of 120 ml/hr and 0,17 $m^3$ (6.0 SCFH) respectively. The vaporized aldehyde and hydrogen were fed to the Berty reactor operated at a pressure of 5,2 bar (60 psig) and temperature of 180°C. The product vapor was withdrawn from the reactor and fed to a brine cooled condenser and the gas and liquid separated. Both liquid product and vent gas were collected and analyzed by a gas chromatograph. The reaction product had the following composition: 6.62 weight percent butyraldehyde, 91.21 weight percent butanol, 1.47 weight percent butyl butyrate and 0.27 weight percent 2-ethylhexanol. No ethers or hydrocarbon gases were formed.

**Claims**

1. A heterogeneous vapor phase process for the catalytic hydrogenation of aldehyde(s) containing 2 to 8 carbon atoms to the corresponding alcohol(s) by contacting a vaporous stream of aldehyde(s) and a hydrogen containing gas with a solid catalyst comprising a reduced mixture of CuO and ZnO at elevated temperatures characterized that the reaction is carried out at a temperature of between 110° and 180°C, a pressure of between 2,4 and 11,5 bar (20 and 150 psig) and a space velocity of between 500 and 4000 $hr^{-1}$.

2. The vapor phase process according to claim 1, wherein said aldehyde is propionaldehyde.

3. The vapor phase process according to claim 1, wherein said aldehyde is a butyraldehyde.

4. The vapor phase process according to claim 1, wherein said aldehyde is a mixture of 2-methyl-butyraldehyde and n-butyraldehyde or a mixture of iso-butyraldehyde and n-butyraldehyde.

5. The vapor phase process according to claim 1, wherein said aldehyde is n-pentanal or 2-ethyl-hexanal.

6. The vapor phase process according to claim 1, wherein said aldehyde is 2-ethyl-3-propylacrolein.

7. The vapor phase process according to any one of the preceding claims, wherein the temperature is between 130° and 170°C.

8. The vapor phase process according to any one of the preceding claims, wherein the pressure is between 4,5 and 8 bar (50 and 100 psid).

9. The vapor phase process according to any one of the preceding claims, wherein the mixture of CuO and ZnO, before reduction, contains from 10 to 70, preferably 20 to 40 percent by weight CuO and 90 to 30, preferably 60 to 80 percent by weight ZnO.

10. The vapor phase process according to any one of the preceding claims, wherein the space velocity is between 1000 and 4000 $hr^{-1}$.

**Patentansprüche**

1. Heterogenes Dampfphasen-Verfahren zur katalytischen Hydrierung von Aldehyd(en) mit 2 bis 8 Kohlenstoffatomen zu dem (den) entsprechenden Alkohol(en) durch Berührung eines dampfförmigen Stromes des (der) Aldehyds(e) und eines wasserstoffhaltigen Gases mit einem festen Katalysator, der eine reduzierte Mischung aus CuO und ZnO enthält, bei erhöhten Temperaturen, dadurch gekennzeichnet, daß die Reaktion bei einer Temperatur zwischen 110 und 180°C, einem Druck zwischen 2,4 und 11,5 bar (20 und 150 psig) und einer Raumgeschweindigkeit zwischen 500 und 4000 $hr^{-1}$ durchgeführt wird.

2. Dampfphasen-Verfahren nach Anspruch 1, dadurch gekennzeichnet, daß der Aldehyd Propionaldehyd ist.

3. Dampfphasen-Verfahren nach Anspruch 1, dadurch gekennzeichnet, daß der Aldehyd Butyraldehyd ist.

4. Dampfphasen-Verfahren nach Anspruch 1, dadurch gekennzeichnet, daß der Aldehyd eine Mischung aus 2-Methylbutyraldehyd und n-Butyraldehyd oder eine Mischung aus Isobutyraldehyd und n-Butyraldehyd ist.

5. Dampfphasen-Verfahren nach Anspruch 1, dadurch gekennzeichnet, daß der Aldehyd n-Pentanal oder 2-Äthylhexanal ist.

6. Dampfphasen-Verfahren nach Anspruch 1, dadurch gekennzeichnet, daß der Aldehyd 2-Äthyl-3-propylacrolein ist.

7. Dampfphasen-Verfahren nach irgendeinem der vorhergehenden Ansprüche, dadurch gekennzeichnet, daß die Temperatur zwischen 130 und 170°C liegt.

8. Dampfphasen-Verfahren nach irgendeinem der vorhergehenden Ansprüche, dadurch gekennzeichnet, daß der Druck zwischen 4,5 und 8 bar (50 und 100 psig) liegt.

9. Dampfphasen-Verfahren nach irgendeinem der vorhergehenden Ansprüche, dadurch gekennzeichnet, daß die Mischung aus CuO und ZnO vor der Reduktion 10 bis 70, vorzugsweise 20 bis 40, Gew.-% CuO und 90 bis 30, vorzugsweise 60 bis 80, Gew.-% ZnO enthält.

10. Dampfphasen-Verfahren nach irgendeinem der vorhergehenden Ansprüche, dadurch gekennzeichnet, daß die Raumgeschwindigkeit zwischen 1000 und 4000 $hr^{-1}$ liegt.

## Revendications

1. Procédé en phase vapeur hétérogène pour l'hydrogénation catalytique d'un ou plusieurs aldéhydes contenant 2 à 8 atomes de carbone en l'alcool ou les alcools correspondants par contact d'un courant à l'état de vapeur d'un ou plusieurs aldéhydes et d'un gaz contenant de l'hydrogène, avec un catalyseur solide comprenant un mélange réduit de CuO et de ZnO à des températures élevées, caractérisé en ce que la réaction est conduite à une température comprise entre 110 et 180°C et à une pression comprise entre 2,4 et 11,5 bars (20 à 150 livres par pouce carré au manomètre) et à une vitesse spatiale de 500 à 4000 $h^{-1}$.

2. Procédé en phase vapeur suivant la revendication 1, caractérisé en ce que l'aldéhyde est le propionaldéhyde.

3. Procédé en phase vapeur suivant la revendication 1, caractérisé en ce que l'aldéhyde est un butyraldéhyde.

4. Procédé en phase vapeur suivant la revendication 1, caractérisé en ce que l'aldéhyde est un mélange de 2-méthylbutyraldéhyde et de n-butyraldéhyde ou un mélange d'isobutyraldéhyde et de n-butyraldéhyde.

5. Procédé en phase vapeur suivant la revendication 1, caractérisé en ce que l'aldéhyde est le n-pentanal ou le 2-éthylhexanal.

6. Procédé en phase vapeur suivant la revendication 1, caractérisé en ce que l'aldéhyde est la 2-éthyl-3-propylacroléine.

7. Procédé en phase vapeur suivant l'une quelconque des revendications précédentes, caractérisé en ce que la température est comprise entre 130 et 170°C.

8. Procédé en phase vapeur suivant l'une quelconque des revendications précédentes, caractérisé en ce que la pression est comprise entre 4,5 et 8 bars (50 et 100 livres par pouce carré au manomètre).

9. Procédé en phase vapeur suivant l'une quelconque des revendications précédentes, caractérisé en ce que le mélange de CuO et de ZnO, avant la réduction, contient 10 à 70, de préférence 20 à 40% en poids de CuO et 90 à 30, de préférence 60 à 80% en poids de ZnO.

10. Procédé en phase vapeur suivant l'une quelconque des revendications précédentes, caractérisé en ce que la vitesse spatiale est comprise entre 1000 et 4000 $h^{-1}$.